# EUROPEAN PATENT SPECIFICATION

(11) **EP 3 231 389 B1**
(45) Date of publication and mention of the grant of the patent: **19.12.2018**
(21) Application number: 17173255.5
(22) Date of filing: 01.03.2012
(51) Int. Cl.: A61B 46/00, A61B 46/20, A61M 1/00

(54) **SURGICAL DRAPE WITH SELECTIVELY DETACHABLE BARRIER**
CHIRURGISCHES ABDECKTUCH MIT SELEKTIV ABNEHMBARER BARRIERE
DRAP CHIRURGICAL DOTÉ D'UNE BARRIÈRE SÉLECTIVEMENT DÉTACHABLE

(30) Priority: 02.03.2011 US 201161448250 P
(43) Date of publication of application: 18.10.2017
(62) Divisional of application: 12751757.1
(73) Proprietor: Medline Industries, Inc., Mundelein, IL 60060-4486 (US)
(72) Inventor: LOCKWOOD, Robert, Libertyville, IL 60048 (US); PRESKILL, David, Highland Park, IL 60035 (US)
(74) Representative: Kuhnen & Wacker Patent- und Rechtsanwaltsbüro PartG mbB

(56) References cited:
- WO-A1-95/10986
- SU-A1- 445 412
- US-A- 3 698 395
- US-A- 4 899 762
- US-A- 6 007 564
- US-A1- 2004 103 903
- US-A1- 2009 277 460

## Description

### FIELD OF THE INVENTION

The present invention relates generally to surgical drapes.

### BACKGROUND OF THE INVENTION

Surgical and other medical treatment procedures require a sterile field to avoid infection. To that end, sterile surgical drapes were developed. Surgical drapes are employed to keep the surgical site sterile from any non-sterile surfaces and environments in part to reduce the infection potential to the patient. Maintaining sterility in and about the surgical site is not only a concern during the operation or procedure, but also after the operation or procedure is complete and the patient is recovering.

Surgical drapes can be used in high volume surgeries. As used herein, the term "high volume surgery" means a surgery, operation or procedure which results in significant fluid loss, particularly abdominal procedures and operations, or surgical procedures which require significant irrigation of the surgical site. For instance, a Cesarean section (C-section) operation is a high volume surgery because it results in a large volume of fluid loss by the patient. The surgical drapes used in C-section procedures contain pockets or pouches made from folds in the surgical drape or some other impermeable liner.

US 4 899 762 A teaches a surgical drape comprising a patient side and a surgeon side and an adhesive material on the patient side. The surgical drape further comprises an antimicrobial substance on a surface of the surgical drape.

US 2009/277460 A1 teaches a surgical drape comprising a window for access to the operating site. An adhesive allows sheets of the drape to be separated by peeling. The window is shared between the sheets.

### SUMMARY OF THE INVENTION

The surgical drape of the invention comprises the features of claim 1. Further improvements are subject to the dependent claims.

### BRIEF DESCRIPTION OF THE DRAWINGS

The foregoing and other advantages of the invention will become apparent upon reading the following detailed description and upon reference to the drawings.
FIG. 1 is a cross section view of a prior art surgical drape.
FIG. 2 is a top view of a prior art surgical drape as applied to a patient.
FIG. 3 is a top view of one embodiment of the surgical drape according to the present invention.
FIG. 4 is a top view of an alternate surgical drape according not forming part of the present invention.
FIG. 5 is a cross section view of the surgical drape as depicted in FIG. 4.
FIG. 6 is a magnified view of the fenestrated ring in FIG. 4.
FIG. 7 is a top view of an alternate embodiment of the surgical drape according to the present invention.
FIG. 8 is a flowchart demonstrating the use of the surgical drape.

### DESCRIPTION OF ILLUSTRATIVE EMBODIMENTS

The present technology is directed to a surgical drape that has a removable portion that is left behind with the patient for a predetermined period of time.

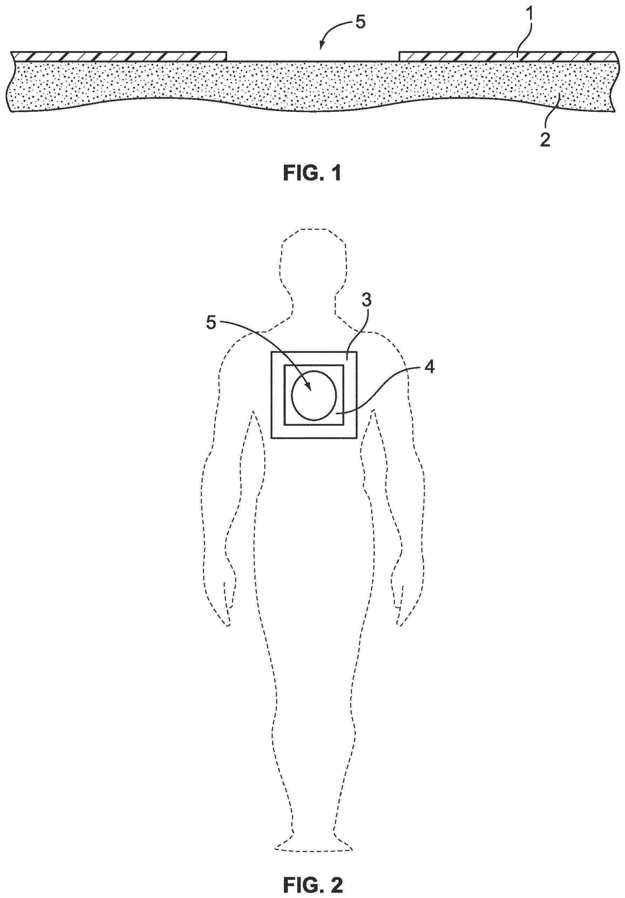

There are many concerns doctors address for their patients when dealing with any type of invasive surgery. Infection, scarring, and physical appearance are just some of these issues. Currently, in operating rooms across the country, surgeons use a surgical drape which forms a barrier between the outside elements and the patient's skin during surgery. The current standard surgical drape surrounds the anticipated surgical field, typically called a fenestration, and is removed prior to closure of the incision. Figure 1 depicts a cross section of this current standard surgical drape **1** surrounding the surgical site **5** as it lies atop the patient's skin **2.** As illustrated in Figure 2, this current standard surgical drape surrounds the surgical field **5** and consists of two materials: plastic **4,** such as a film and generally known as an incise and a barrier fabric such as a non-woven material or other paper **3.**

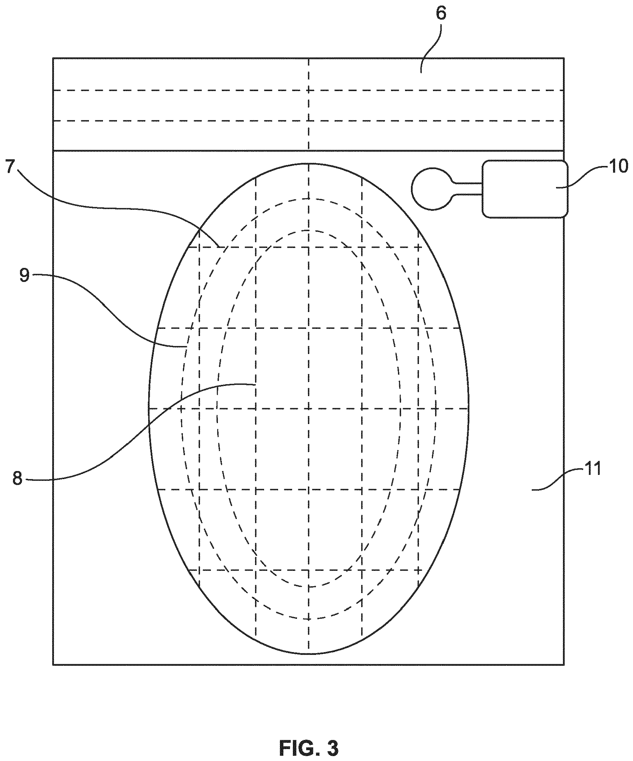

A new surgical drape is provided herein. Once the patient's skin has been thoroughly cleaned, the surgical drape can be affixed to the patient's skin by an adhesive on the surgical drape's surface. As illustrated in Figure 3, the surgical drape comprises a thin and plastic material **11** and can be translucent. This translucent material enables the surgeon to make the incision directly through the surgical drape without screening the surgical field from the surgeon's view while still providing protection for the actual incision from the outside elements. The plastic material may have antimicrobial characteristics to reduce infection potential and may be left in place post-operatively. In one embodiment, the surface of the material can be coated with any antimicrobial substance to provide the benefit of carrying the sterility of the surgical field through the post-operative healing time. In other embodiments, the antimicrobial characteristic is incorporated into the plastic/incise material. Said another way, the antimicrobial properties of the surgical drape can be due to the surgical drape's material or an antimicrobial substance added to the surgical drape's surface. Antimicrobial substances include, but are not limited to, germicides, antibiotics, antibacterials, antivirals, antifungals, antiprotozoals and antiparasites. Specifically, silver ions or compounds, chlorhexidine, iodine or iodine based, triclosan, bacitracin, clindamycin, erythromycin, metronidazole, mupirocin, neomycin, retamapulin, polymyxin B, mupirocin, fusidic acid, gentamicin, or combinations thereof are contemplated. The antimicrobial substance can be provided in solutions, pastes, suspensions, gels or films. An advantage of this embodiment of the surgical drape is the decrease in the chance of post-operative infection.

The surgical drape may be equipped with factory-made or pre-made perforations. These perforations may be presented in various patterns so that the surgical drape may be configured for use by different surgeons or for different types of procedures. Figure 3 demonstrates how these perforations can be integrated into the surgical drape as well as a glue packet 10 affixed to the drape which can assist in surgical field closure. These perforations provide the surgeon the option of selecting (1) the size of the drape to be used in the operation and (2) the size of the surgical field. Different operations require various sizes of surgical drapes and sterile fields. By tailoring the size of the surgical drape to the operation or procedure, the surgeon can reduce the risk of dislodging the drape and compromising any sterile field. The factory perforations at the termination of the surgical drape **6** allow the surgeon the flexibility to change the size of the drape. The factory perforations placed in a grid configuration - horizontally **7** and/or vertically **8** across the surgical site of the surgical drape allow the surgeon the flexibility of defining a square or rectangular drape or surgical field of various sizes. The surgeon need not use these perforations to define the surgical field if he/she desires to make the incision directly through the surgical drape.

Further, these perforations through the surgical site can aid the surgeon during the closure of the surgical field. If the surgeon chooses to remove the drape immediately after surgery, these perforations will allow for partial or sequential removal of the plastic / adherent drape material so that the surgeon can still utilize the drape to aid in the closure during surgery. Also, because the perforated portions of the drape can be removed sequentially, the drape can accommodate different styles of closure. Therefore, the surgeon's preferred style of closure, be it continuous suturing, individual sutures, and/or staples, for example, need not be altered to take advantage of the many other benefits provided by the present drape, including aligning the incision. In addition to the grid perforation configuration, a second pattern of perforations, for example, a concentric oval pattern 9, will allow the surgeon to choose how much of the drape material is left in place over and around the surgical site both during the operation and post-operatively. Numerous other patterns are contemplated, including, but not limited to an animal or cartoon character pattern.

The surgical drape may also be equipped with markings; the markings, used either in conjunction with or instead of the factory perforations, will allow the surgeon to more accurately line up the patient's skin during the closure of the surgical site. This feature will allow for a more aesthetic and symmetrical closing, therefore minimizing the chances for any type of disfigurement to the patient. This feature is especially valuable when the surgical site necessitates an incision into skin already under tension due to an underlying growth or edema. For example, these markings would be especially useful in cases where the surgeon is excising a tumor or performing a C-section operation. During a C-section the skin on the patient's abdomen is stretched and can therefore pose a challenge to the surgeon attempting to close the abdomen after birth. With the current invention in place, the surgeon can utilize the markings, or perforations, on the surgical drape to guide alignment and complete the closure with little worry as to symmetry or positioning.

Also, because the surgical drape can remain in place post-operatively, the surgical drape's adhesive properties can be used to aid in keeping the incision not only sterile, but also closed. Due to this benefit, the surgeon may be able to utilize a surgical tape or glue to hold together major incisions, where surgery using conventional surgical drapes may have required sutures and staples. This provides a benefit to the patient as it will greatly reduce post-operative scarring. Of course, more typical surgical closure devices may be used, including, but not limited to adhesive strips, paper tape, staples, sutures or surgical glue.

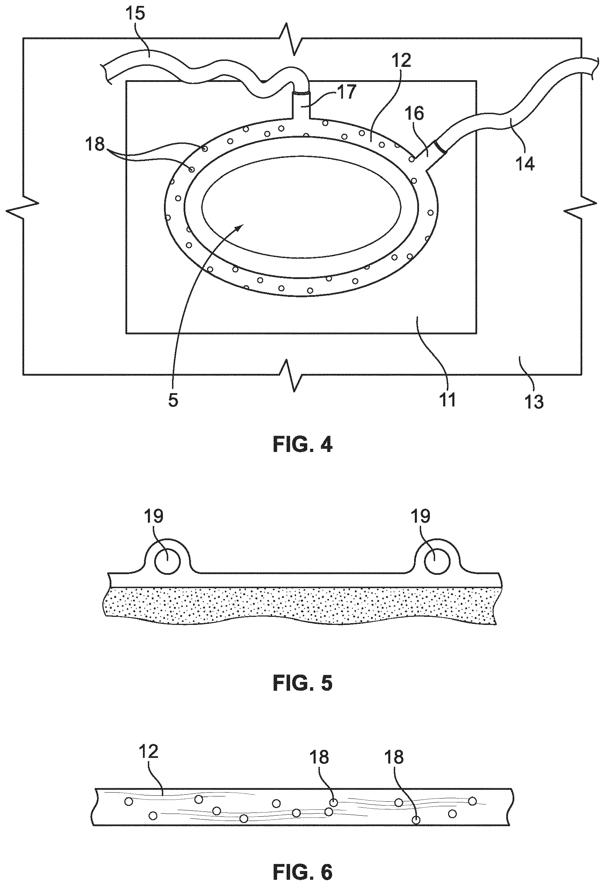

Figures 4-6 depict an exemplary surgical drape adapted for use in high volume surgeries. Specifically, this surgical drape would be well-suited for use in conjunction with C-section operations. During a C-section the patient typically releases an enormous amount of fluid (amniotic and blood) at the moment of infant delivery. Fenestrated drapes using an adhesive plastic section surrounded by a paper section, are unable to stay fixed on patients because of the high fluid output coupled to the necessary handling of the surgical drape during the procedure. The confluence of these events creates a separation between the surgical drape and patient. Invariably fluid leaks out and the surgeons and floor are covered with this amniotic-blood slurry. This fluid leak constitutes a safety problem for the patient and the hospital staff.

Figure 4 shows the exemplary surgical drape designed for high-volume surgeries but not forming part of the present invention. This surgical drape has an adhesive plastic section **11** surrounded by a paper section **13,** and also including a fenestrated ring **12** which is hollow or tubular and has one or more openings. The fenestrated ring **12** surrounds the surgical field **5,** and is under the plastic section **11.** In use, the fenestrated ring is place on the patient's skin, below the plastic section **11** of the surgical drape. The fenestrated ring **12** has a port **16** for attachment to a vacuum tube **14,** to provide suction within the ring. The vacuum within the fenestrated ring **12** will hold the surgical drape in place and evacuate any fluids with which the fenestrations **18** comes into contact. This will greatly reduce leaking of fluid, keeping the fluid away from the floor and the hospital staff. The fenestrated ring **12** may also include a second port **17** for attachment of a hand held suction tube **15** for use during the procedure.

Figure 5 depicts the lumen **19** of the fenestrated ring which would carry the evacuated fluids away from the surgical site towards the vacuum source.

Figure 6 shows the fenestrated ring **12** magnified to illustrate the fenestrations 18 in the fenestrated ring **12** which provide the avenue by which the fluids are evacuated.

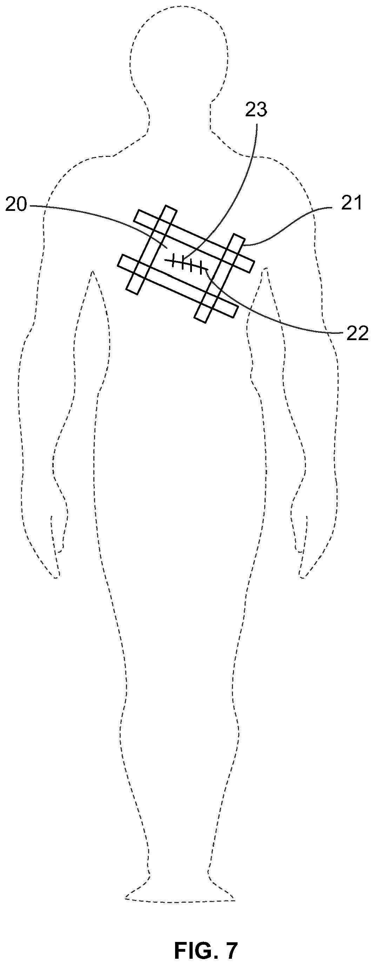

Figure 7 depicts the surgical drape **20** left on a patient during the post-operative recovery period. The surgical drape can be further secured to the patient using tape **21** or other means. The incision **22** is shown to be made through the drape and then the wound is closed by suturing or stapling **23** through the drape.

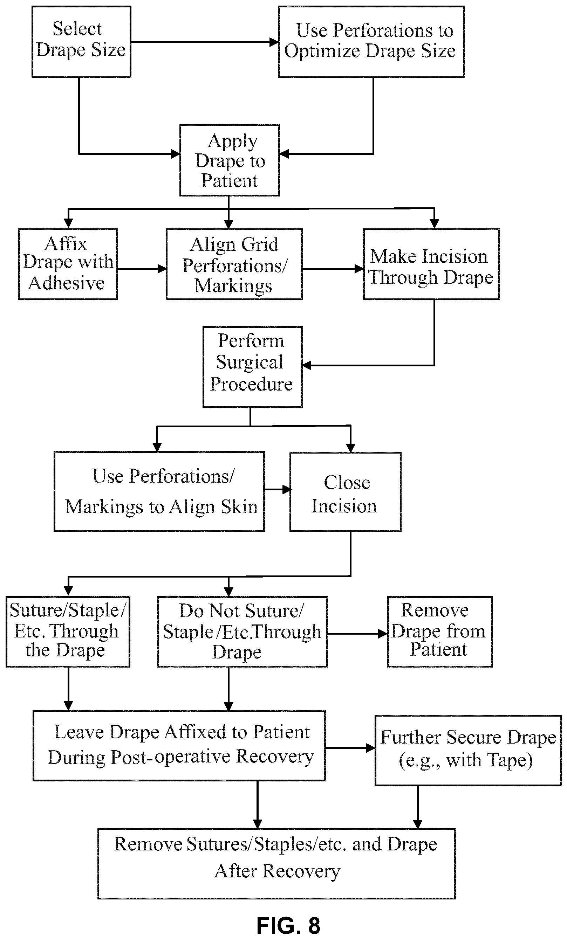

Figure 8 portrays an exemplary decision-making flowchart demonstrating how the surgical drape can used by the surgeon, including certain decision points.

In another preferred embodiment, the adhesive plastic section may contain excess or redundant material. The plastic material can be formed into a ridge or barrier adapted to prevent fluid from overflowing from the surgical site. This ridge or barrier draping configuration prevents separation of the surgical drape from the patent's skin due to tension and movement of the patient's skin or the plastic section where it is adhered. The ridge or barrier draping configuration may be used with the fenestrated tube, or instead of the fenestrated tube. If the excess material forming a ridge or barrier is used in conjunction with the fenestrated ring, the ridge or barrier will hold the fluid in place until the fenestrated ring can evacuate the fluid.

The present surgical drapes also provide a branding opportunity. The logo of either the hospital providing the surgical drape or the signature of the surgeon can be placed directly onto the surgical drape itself. This can provide the doctor the benefit of "signing his work" and/or the hospital the benefit of a new and interesting form of advertising; the first thing many visitors say to a recovering surgical patient is "let me see your incision". With the present surgical drape in place, that simple request can lead to an advertisement opportunity for the surgeon and/or the hospital. Additional branding, including, but not limited to cartoon characters or other objects are contemplated in the present surgical drapes.

Due to the many ways the current invention can be utilized, a "surgical kit" may be assembled for retail sale. This kit would include not only the aforementioned surgical drape, but will also include the surgical tape and/or surgical glue discussed above as part of a more aesthetic closing technique. The surgical tape may be made of a similar material to the surgical drape, or a currently used paper tape, and would adhere to the surgical drape more strongly than to the skin. Usage of this would allow for "cross-taping" over the incision where the surgical drape was left on post-operatively, therefore increasing the chances that the closure can be made suture or staple free, yet retain the time advantage of staples without the physical scarring associated with them. The surgical glue, such as or similar to Octylseal™, would adhere the skin to itself, eliminating the need for top layer suturing or stapling. It may be provided in a single use applicator packaged in a blister pouch. The applicator may be a crushable glass ampule contained within a plastic vial with attached applicator tip. The surgical glue provides the same benefits as the tape, where closure and/or scarring reduction is concerned. Providing a variety of surgically useful devices in one surgical kit not only provides convenience for the surgeon and his staff, but would also aid in the surgeon's ability to provide better patient care.

While the present invention has been described with reference to one or more particular embodiments, those skilled in the art will recognize that many changes may be made thereto without departing from the scope of the present invention. Each of these embodiments and obvious variations thereof is contemplated as falling within the scope of the invention, which is set forth in the claims.

## Claims

1. A surgical drape (6) comprising:
a patient side and a surgeon side;
an adhesive material on the patient side;
an antimicrobial substance on a surface of the surgical drape (6); and
perforations (9) in a concentric configuration;
**characterized in that** the surgical drape (6) further comprises perforations (7, 8) in a grid configuration.

2. The surgical drape (6) of claim 1, further comprising second perforations, said second perforations disposed to permit portions of an outer edge of the surgical drape (6) to be selectively removed from a remaining portion of the surgical drape (6) to thereby change a size of the surgical drape (6).

3. The surgical drape (6) of claim 1 or 2, wherein the concentric perforations (9) are concentric oval perforations (9).

4. The surgical drape (6) of any one of claims 1 to 3, wherein the perforations (7, 8) in a grid configuration are at least partially overlapping with the perforations (9) in a concentric configuration.

5. The surgical drape (6) of any of the preceding claims wherein a portion (20) of the surgical drape (6) is configured to be retailed post-operatively on a patient after closure of an incision (22).

6. A surgical kit, comprising:
the surgical drape (6) of claim any one of the preceding claims, and
a surgical closure device.

7. The surgical kit of claim 6, where the surgical closure device is selected from the group consisting of adhesive strips, paper tape, staples (23), sutures, and surgical glue.

## Patentansprüche

1. OP-Abdecktuch (6), aufweisend:
eine Patientenseite und eine Operateurseite;
ein Haftmaterial auf der Patientenseite;
eine antimikrobielle Substanz auf einer Oberfläche des OP-Abdecktuchs (6); und
Perforationen (9) in einer konzentrischen Anordnung;
**dadurch gekennzeichnet, dass** das OP-Abdecktuch (6) ferner Perforationen (7, 8) in einer Gitteranordnung aufweist.

2. OP-Abdecktuch (6) nach Anspruch 1, ferner aufweisend zweite Perforationen, wobei die zweiten Perforationen so angeordnet sind, dass sie es Abschnitten einer äußeren Kante des OP-Abdecktuchs (6) ermöglichen, selektiv von einem verbleibenden Abschnitt des OP-Abdecktuchs (6) entfernt zu werden, um dadurch eine Größe des OP-Abdecktuchs (6) zu verändern.

3. OP-Abdecktuch (6) nach Anspruch 1 oder 2, wobei die konzentrischen Perforationen (9) konzentrische ovale Perforationen (9) sind.

4. OP-Abdecktuch (6) nach einem der Ansprüche 1 bis 3, wobei sich die Perforationen (7, 8) in einer Gitteranordnung zumindest teilweise mit den Perforationen (9) in einer konzentrischen Anordnung überlappen.

5. OP-Abdecktuch (6) nach einem der vorgenannten Ansprüche, wobei ein Abschnitt (20) des OP-Abdecktuchs (6) so konfiguriert ist, dass er postoperativ nach dem Schließen eines Einschnitts (22) wieder über den Patienten gezogen wird.

6. Chirurgie-Kit, aufweisend:
das OP-Abdecktuch (6) nach einem der vorgenannten Ansprüche, und
eine chirurgische Schließvorrichtung.

7. Chirurgie-Kit nach Anspruch 6, wobei die chirurgische Schließvorrichtung aus der Gruppe ausgewählt ist, die aus Klebestreifen, Papierband, Klammern (23), Nähten und chirurgischem Klebstoff besteht.

## Revendications

1. Drap chirurgical (6) comprenant :
un côté patient et un côté chirurgien ;
un matériau adhésif sur le côté patient ;
une substance antimicrobienne sur une surface du drap chirurgical (6) ; et des perforations (9) selon une configuration concentrique ;
**caractérisé en ce que** le drap chirurgical (6) comprend en outre des perforations (7, 8) dans une configuration de grille.

2. Drap chirurgical (6) selon la revendication 1, comprenant en outre des secondes perforations, lesdites secondes perforations disposées pour permettre que des portions d'un bord extérieur du drap chirurgical (6) soient sélectivement retirées d'une partie restante du drap chirurgical (6) pour ainsi changer une taille du drap chirurgical (6).

3. Drap chirurgical (6) selon la revendication 1 ou 2, dans lequel les perforations concentriques (9) sont des perforations concentriques ovales (9).

4. Drap chirurgical (6) selon l'une quelconque des revendications 1 à 3, dans lequel les perforations (7, 8) dans une configuration de grille chevauchent au moins partiellement les perforations (9) dans une configuration concentrique.

5. Drap chirurgical (6) selon l'une quelconque des revendications précédentes dans lequel une partie (20) du drap chirurgical (6) est configurée pour être utilisée en post-opératoire sur un patient après la fermeture d'une incision (22).

6. Kit chirurgical, comprenant :
le drap chirurgical (6) selon l'une quelconque des revendications précédentes, et
un dispositif de fermeture chirurgical.

7. Kit chirurgical selon la revendication 6, dans lequel le dispositif de fermeture chirurgical est sélectionné dans le groupe constitué de bandelettes adhésives, de bande de papier, d'agrafes (23), de sutures, et de colle chirurgicale.
